# EUROPEAN PATENT SPECIFICATION

(11) **EP 4 501 289 B1**
(45) Date of publication and mention of the grant of the patent: **14.01.2026**
(21) Application number: 24187850.3
(22) Date of filing: 10.07.2024
(51) Int. Cl.: A61F 5/44, A61F 5/451, A61F 5/455

(54) **MODULAR SUCTION UNIT FOR A URINARY RELIEF SYSTEM**
MODULARE SAUGEINHEIT FÜR EIN HARNENTLASTUNGSSYSTEM
UNITÉ D'ASPIRATION MODULAIRE POUR SYSTÈME DE SOULAGEMENT URINAIRE

(30) Priority: 02.08.2023 US 202318364383
(43) Date of publication of application: 05.02.2025
(73) Proprietor: Rockwell Collins, Inc., Cedar Rapids, IA 52498 (US)
(72) Inventor: KUCZEK, Andrzej E., Bristol, 06010 (US); KHAKPOUR, Yasmin, South Windsor, 06074 (US); PEARSON, Matthew R., Hartford, 06106 (US)
(74) Representative: Dehns

(56) References cited:
- EP-A1- 3 977 967
- EP-A1- 4 431 065
- US-A1- 2004 176 746
- US-B2- 7 135 012
- US-B2- 7 448 288

## Description

### FIELD

The present disclosure generally relates urinary relief systems, and more specifically, to pressurized urinary relief systems for pilots.

### BACKGROUND

Aircrew often need to urinate multiple times during flights without removing restraint systems and flight equipment. Current mission profiles and air refueling abilities have led to longer flight times for aircrew. As a result, some aircrew, especially female aircrew, resort to ingesting fewer liquids to reduce the need to urinate, resulting in dehydration. A safe, reliable, and effective system to provide aircrew, especially female aircrew, the capability of bladder relief during flight is sought. EP4431065A1 and EP3977967A1, upon which the preamble of the claim 1 is based, relate to urinary relief systems.

### SUMMARY

A control head for a modular control unit of a urinary relief system is disclosed herein. The control head includes a pressurized gas source input, a toggle configured to activate a pressurized gas source coupled to the pressurized gas source input, a first connector fluidly coupled to the pressurized gas source input and configured to allow a first gas from the pressurized gas source input to flow through the control head, and a second connector fluidly coupled to the first connector and configured to allow a second gas to pass through the control head.

In various embodiments, the control further includes a valve coupled to the pressurized gas source input, wherein the first connector is fluidly coupled to the pressurized gas source input through the valve. In various embodiments, the toggle is further configured to open the valve. In various embodiments, the control head further includes a first connector on the first connector, and a second connector on the second connector, the second connector configured to engage the first connector.

In various embodiments, the first connector includes a first rail and the second connector includes a second rail that is configured to engage the first rail. In various embodiments, the first connector includes a first threading and the second connector includes a second threading that is configured to engage the first threading. In various embodiments, the first connector includes a first snap connector and the second connector includes a second snap connector that is configured to engage the first snap connector.

Also disclosed herein is a modular control unit for a urinary relief system. The modular control unit includes a first pressurized gas source, a pressure regulator configured to regulate a pressure of the first pressurized gas source, an ejector coupled to the pressure regulator and an output, an ejector head housing the pressure regulator and the ejector, and a first control head coupled to the ejector head housing. The first control head includes a first pressurized gas source input configured to receive the first pressurized gas source, a first toggle configured to activate the first pressurized gas source, a first connector fluidly coupled to the first pressurized gas source input and fluidly coupled to the ejector head housing, the first connector configured to allow a first gas from the first pressurized gas source input to flow through the first control head and into the ejector head housing, and a second connector fluidly coupled to the first connector and configured to allow a second gas to pass through the first control head.

In various embodiments, the modular control unit further includes a second pressurized gas source, and a second control head coupled to the first control head. The second control head includes a second pressurized gas source input configured to receive the second pressurized gas source, a second toggle configured to activate the second pressurized gas source, and a first connector fluidly coupled to the second pressurized gas source input and fluidly coupled to the first control head, the first connector configured to allow the second gas from the second pressurized gas source input to flow through the second control head and into the first control head through the second connector of the first control head.

In various embodiments, the second connector of the first control head is configured to receive the first connector of the second control head. In various embodiments, the second connector of the first control head includes a first rail and the first connector of the second control head includes a second rail that is configured to engage the first rail. In various embodiments, the modular control unit further includes a housing coupled to the ejector head housing and the second control head, the housing being configured to house the first pressurized gas source and the second pressurized gas source.

In various embodiments, the modular control unit further includes a first housing coupled to the ejector head housing and the first control head, the first housing configured to house the first pressurized gas source, and a second housing coupled to the first housing and the second control head, the second housing configured to house the second pressurized gas source. In various embodiments, the first control head includes a first valve coupled to the first pressurized gas source input, wherein the first connector is fluidly coupled to the first pressurized gas source input through the first valve, wherein the first toggle is further configured to open the first valve.

Also disclosed herein is a urinary relief system that includes a human interface device, a storage device coupled to the human interface device, and a modular control unit coupled to the storage device. The modular control unit includes a first pressurized gas source, a pressure regulator configured to regulate a pressure of the first pressurized gas source, an ejector coupled to the pressure regulator and an output, an ejector head housing the pressure regulator and the ejector, a first control head coupled to the ejector head housing. The first control head includes a first pressurized gas source input configured to receive the first pressurized gas source, a first toggle configured to activate the first pressurized gas source, a first connector fluidly coupled to the first pressurized gas source input and fluidly coupled to the ejector head housing, the first connector configured to allow a first gas from the first pressurized gas source input to flow through the first control head and into the ejector head housing, and a second connector fluidly coupled to the first connector and configured to allow a second gas to pass through the first control head.

In various embodiments, the modular control unit further includes a second pressurized gas source, and a second control head coupled to the first control head. The second control head includes a second pressurized gas source input configured to receive the second pressurized gas source, a second toggle configured to activate the second pressurized gas source, and a first connector fluidly coupled to the second pressurized gas source input and fluidly coupled to the first control head, the first connector configured to allow the second gas from the second pressurized gas source input to flow through the second control head and into the first control head through the second connector of the first control head.

In various embodiments, the second connector of the first control head is configured to receive the first connector of the second control head. In various embodiments, the urinary relief system further includes a housing coupled to the ejector head housing and the second control head, the housing being configured to house the first pressurized gas source and the second pressurized gas source. In various embodiments, the urinary relief system further includes a first housing coupled to the ejector head housing and the first control head, the first housing configured to house the first pressurized gas source, and a second housing coupled to the first housing and the second control head, the second housing configured to house the second pressurized gas source. In various embodiments, the first control head is slidably coupled to the second control head.

The foregoing features and elements may be combined in any combination, without exclusivity, unless expressly indicated herein otherwise. These features and elements as well as the operation of the disclosed embodiments will become more apparent in light of the following description and accompanying drawings.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the following detailed description and claims in connection with the following drawings. While the drawings illustrate various embodiments employing the principles described herein, the drawings do not limit the scope of the claims.
FIGS. 1A and 1B illustrate a urinary relief system, in accordance with various embodiments.
FIG. 2 illustrates a functional diagram of a urinary relief system, in accordance with various embodiments.
FIGS. 3A, 3B, 3C, and 3D illustrate an integrated pressure regulator and ejector pump, in accordance with various embodiments.
FIG. 4 illustrates an integrated pressure regulator and ejector pump, in accordance with various embodiments.
FIG. 5 illustrates an integrated pressure regulator and ejector pump and modular valve housings connected to pressurized gas sources, in accordance with various embodiments.
FIGS. 6A and 6B illustrate a modular integrated pressure regulator and ejector pump and modular valve housings connected to pressurized gas sources within a housing, in accordance with various embodiments.

### DETAILED DESCRIPTION

The following detailed description of various embodiments herein makes reference to the accompanying drawings, which show various embodiments by way of illustration. While these various embodiments are described in sufficient detail to enable those skilled in the art to practice the disclosure, it should be understood that other embodiments may be realized and that changes may be made without departing from the scope of the disclosure. Thus, the detailed description herein is presented for purposes of illustration only and not of limitation. The steps recited in any of the method or process descriptions may be executed in any order and are not necessarily limited to the order presented. Furthermore, any reference to singular includes plural embodiments, and any reference to more than one component or step may include a singular embodiment or step. Also, any reference to attached, fixed, connected, or the like may include permanent, removable, temporary, partial, full or any other possible attachment option. Additionally, any reference to without contact (or similar phrases) may also include reduced contact or minimal contact. It should also be understood that unless specifically stated otherwise, references to "a," "an" or "the" may include one or more than one and that reference to an item in the singular may also include the item in the plural. Further, all ranges may include upper and lower values and all ranges and ratio limits disclosed herein may be combined.

Disclosed herein, in various embodiments, is a urinary relief system including a human interface, a storage unit, and a control unit. The human interface is configured to collect and pass a liquid (e.g., urine) from a user (e.g., a pilot) to the storage unit. In various embodiments, the human interface may be designed to be worn under clothing (e.g., flight suit) and close to the body of the user and for prolonged periods of time.. In various embodiments, the human interface may be fluidly coupled to the storage unit by a hose and, in various embodiments, a quick connector coupling. The storage unit is configured to collect and store the liquid (e.g., urine). In various embodiments, the storage unit includes an absorbent material configured to store the liquid in a more viscous form, which may less prone to leakage. The various embodiments, the storage unit includes an input fluidly coupled to the human interface device and an output fluidly coupled to the control unit. In various embodiments, the output includes a liquid restrictor so that little to no liquid is passed to the control unit. In various embodiments, the storage unit and the control unit may be coupled by a hose and, in various embodiments, a quick connector coupling.

In various embodiments, the control unit includes an ejector head having an integrated pressure regulator and ejector pump. In various embodiments, the control unit includes an ejector head housing, one or more control heads that are removably coupled to each other, a pressurized gas source (e.g., CO₂) coupled to each pump housing, and, in various embodiments, a housing to protect the pressurized gas sources. In various embodiments, the control unit includes one or more air ejector pumps driven by one or more CO₂ cartridges. In various embodiments, the control unit further includes one or more pressure regulators between the CO₂ cartridges and the one or more ejector pumps. In various embodiments, the control unit includes a single housing that encloses the one or more ejector pumps, the one or more pressure regulators, and the one or more CO₂ cartridges. In various embodiments, the control heads include couplings to allow multiple control heads to be coupled together to increase the capacity of the control unit.

In various embodiments, the control unit provides suction to draw the liquid (e.g., urine) from the human interface into the storage unit. In various embodiments, the one or more pressure regulators reduces the working pressure of the control unit by up to 30 times the pressure of each CO₂ cartridge. In various embodiments, the reduction in pressure may result in an increase to operation run time of the urinary relief system. In various embodiments, the size of the control unit is reduced by integrating the pressure regulator and the ejector into a single ejector head unit. The control until described herein in its various embodiments may provide a compact design, an extended performance time, a modular control unit, and/or lower cost of fabrication.

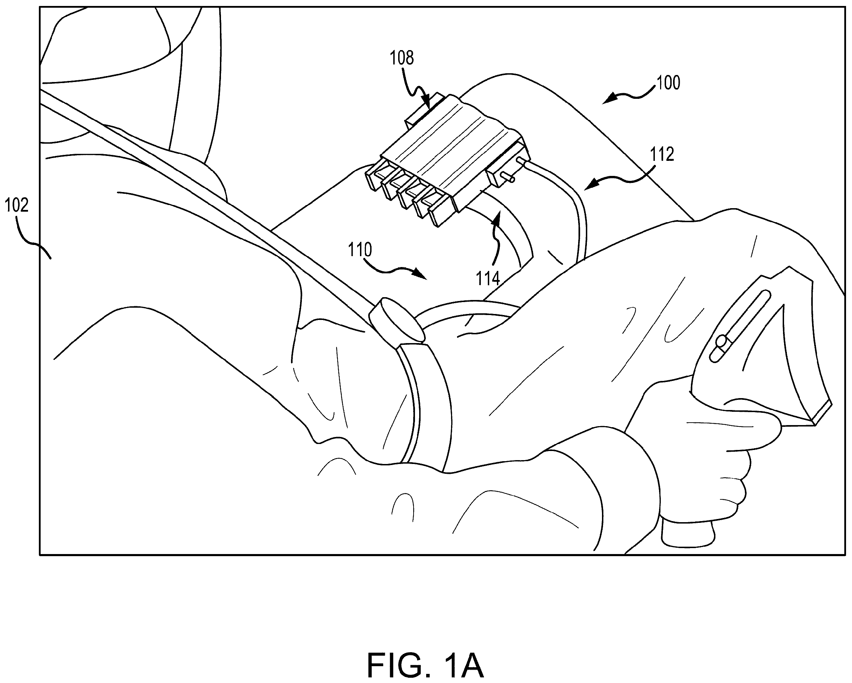

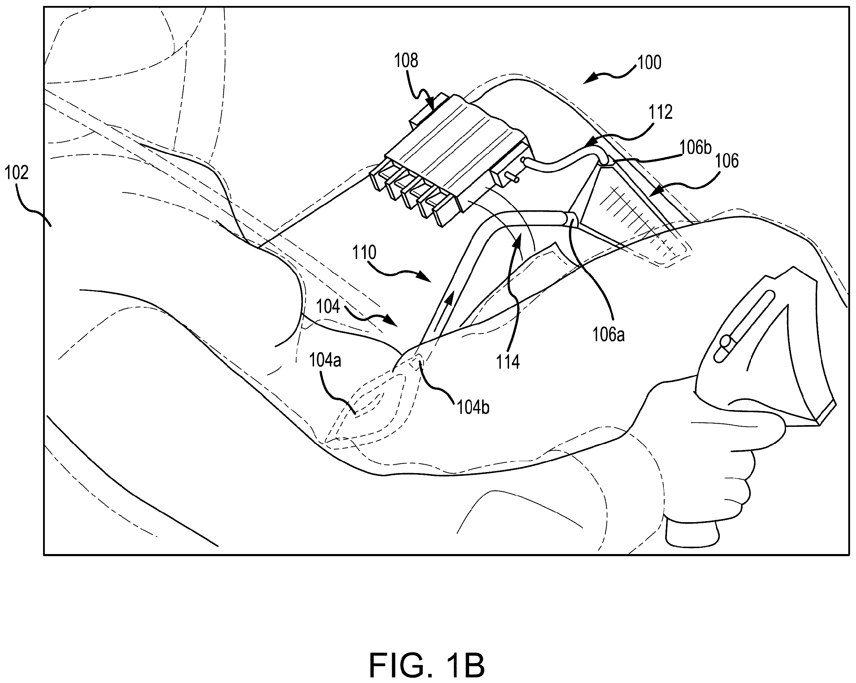

Referring to FIGS. 1A and 1B, a urinary relief system 100 for use by a pilot 102 (e.g., a female pilot) is illustrated, in accordance with various embodiments. Urinary relief system 100 includes a human interface device 104, a storage unit 106, and a control unit 108. Human interface device 104 is configured to be positioned under the clothing (e.g., flight suit, underwear, pants, etc.) and immediately adjacent the body of pilot 102 for use in flight. That is, between the body and clothing of pilot 102 when pilot 102 is in a seated position. In various embodiments, human interface device 104 may form a seal with the body of pilot 102. Human interface device 104 may be any size and/or shape conducive for placement under the clothing of pilot 102 and for collecting the liquid (e.g., urine) while in place. Human interface device 104 includes a collection portion 104a and an outlet port 104b.

Storage unit 106 includes a storage inlet port 106a and a storage outlet port 106b. Outlet port 104b of human interface device 104 is connected to storage inlet port 106a of storage unit 106 by an inlet hose 110 and storage outlet port 106b of storage unit 106 is connected to control unit 108 by an outlet hose 112. In various embodiments, storage unit 106 may be disposable. In various embodiments, control unit 108 and/or storage unit 106 may be secured to pilot 102 (e.g., secured to a leg) by one or more straps 114. Storage unit 106 is configured to receive and trap liquid (e.g., urine) received from storage inlet port 106a and not allow the liquid to exit through storage outlet port 106b.

Control unit 108 creates a motive force that draws the liquid (e.g., urine) from human interface device 104 and into storage unit 106. Air may be drawn, along with the liquid, into storage unit 106 by the vacuum created by control unit 108. In various embodiments, storage unit 106 includes a filter, or flow restrictor, which allows the air to exit storage unit 106 through storage outlet port 106b and keep the liquid secured in storage unit 106. In various embodiments, control unit 108 provides the motive force by creating a vacuum to draw the liquid from human interface device 104 to storage unit 106.

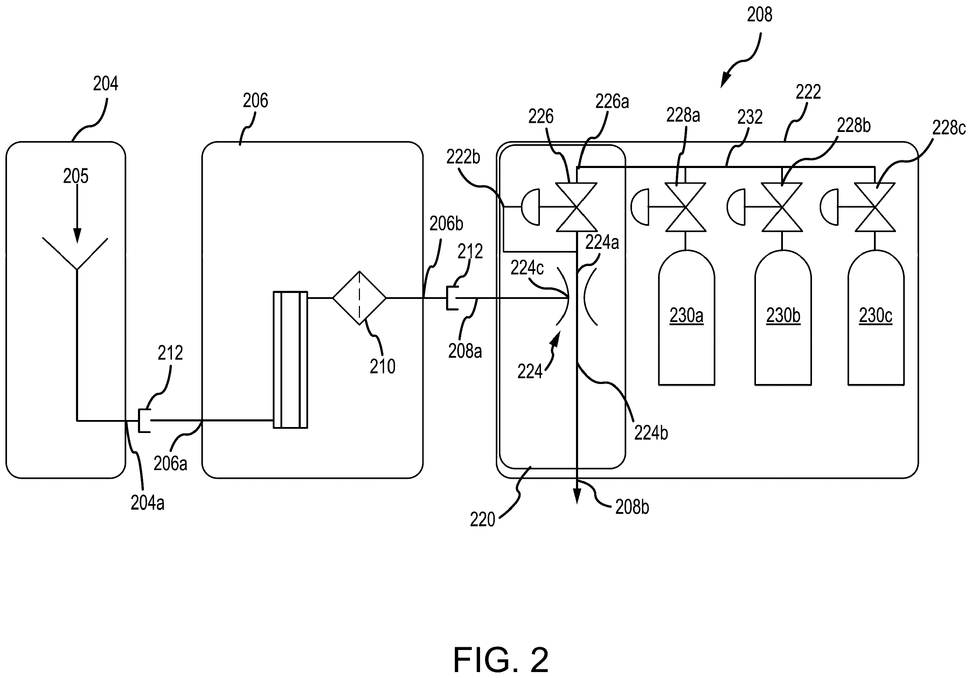

Referring now to FIG. 2, a functional diagram 200 of a urinary relief system is illustrated, in accordance with various embodiments. Functional diagram 200 may be an example of urinary relief system 100 described above with respect to FIGS. 1A and 1B. Functional diagram 200 includes a collection device 204, a storage device 206, and a control device 208. In various embodiments, collection device 204, storage device 206, and control device 208 may be examples of human interface device 104, storage unit 106, and control unit 108, respectively, described above with respect to FIGS. 1A and 1B.

Collection device 204 is configured to collect a liquid 205 (e.g., urine) from a user (e.g., pilot 102). Collection device 204 has an outlet port 204a, storage device 206 has a storage inlet port 206a and a storage outlet port 206b, and control device 208 has a control inlet port 208a and a control outlet port 208b. Outlet port 204a of collection device 204 is configured to transfer the collected liquid from collection device 204 to storage device 206. Outlet port 204a of collection device 204 is connected to storage inlet port 206a of storage device 206. Storage outlet port 206b of storage device 206 is connected to control inlet port 208a of control device 208. In various embodiments, a hose, or tube, may be used to make the connection between outlet and inlet ports. In various embodiments, there is a first quick connector interface 212 between collection device 204 and storage device 206 that allows for quick and simple attachment and detachment of a hose, or tube. In various embodiments, there is a second quick connector interface 214 between storage device 206 and control device 208 that allows for quick and simple attachment and detachment the hose, or tube. In various embodiments, a filter, or flow restrictor 210, is connected to storage outlet port 206b of storage device 206. Flow restrictor 210 prevents liquid (e.g., urine) from passing to control device 208 while allowing air to pass to control device 208.

Control device 208 includes an ejector head 220 and a pressurized gas supply 222. Ejector head 220 includes houses control inlet port 208a, control outlet port 208b, an ejector 224, and a pressure regulator 226. Ejector 224 is coupled to pressure regulator 226, control inlet port 208a, and control outlet port 208b. Pressurized gas supply 222 includes a first valve 228a, a second valve 228b, a third valve 228c, a first CO₂ cartridge 230a, a second CO₂ cartridge 230b, and a third CO₂ cartridge 230c. First CO₂ cartridge 230a is coupled to first valve 228a, second CO₂ cartridge 230b is coupled to second valve 228b, and third CO₂ cartridge 230c is coupled to third valve 228c. First valve 228a, second valve 228b, and third valve 228c are connected in parallel to pressure regulator 226 by a high pressure line 232. That is, each valve 228a, 228b, 228c has an independent high pressure connection to high pressure line 232 that is in turn connected to pressure regulator 226. While the description herein uses CO₂ cartridges, it should be appreciated that any suitable pressurized gas container may be used.

Each CO₂ cartridge 230a, 230b, 230c is sealed and connected to the corresponding valve 228a, 228b, 228c. Each valve 228a, 228b, 228c performs the same function. For example, first valve 228a is configured to puncture the seal of first CO₂ cartridge 230a, in response to an input, allowing the compressed air in first CO₂ cartridge 230a to pass through first valve 228a, into high pressure line 232, and to pressure regulator 226. Second valve 228b is configured to puncture the seal of second CO₂ cartridge 230b, in response to an input, allowing the compressed air in second CO₂ cartridge 230b to pass through second valve 228b, into high pressure line 232, and to pressure regulator 226. Third valve 228c is configured to puncture the seal of third CO₂ cartridge 230c, in response to an input, allowing the compressed air in third CO₂ cartridge 230c to pass through third valve 228c, into high pressure line 232, and to pressure regulator 226.

Pressure regulator 226 has a high pressure inlet 226a and a reduced pressure outlet 226b. Pressure regulator 226 receives the high pressure air from high pressure line 232 at high pressure inlet 226a, reduces the pressure of the air (e.g., CO₂), and outputs the air at a reduced pressure from reduced pressure outlet 226b. The pressure of the air at reduced pressure outlet 226b is about 15 times to about 45 times less than the pressure of the air received at high pressure inlet 226a. In various embodiments, the pressure of the air at reduced pressure outlet 226b is about 25 times to about 35 times less than the pressure of the air received at high pressure inlet 226a. Reducing the pressure of the air by pressure regulator 226 slows the release of the pressurized air (e.g., CO₂) and therefore extends the lifespan of each CO₂ cartridge 230a, 266b, 226c. Adjusting the release pressure (e.g., air pressure at reduced pressure outlet 226b) either up or down may decrease or increase, respectively, the effective lifespan of each CO₂ cartridge 230a, 230b, 230c.

Ejector 224 has an air inlet 224a, an air outlet 224b, and a vacuum port 224c. Air inlet 224a of ejector 224 is connected to reduced pressure outlet 226b of pressure regulator 226. Air outlet 224b is connected to control outlet port 208b which is open to the environment (e.g., cockpit, cabin, etc.). Vacuum port 224c is connected to storage outlet port 206b of storage device 206. Ejector 224 receives pressurized air at air inlet 224a and passes the pressurized air to air outlet 224b to create a vacuum at vacuum port 224c. The vacuum creates a motive force that passes through storage device 206 to collection device 204 and draws, or sucks, air and liquid (e.g., urine) from collection device 204 into storage device 206. A combination of the materials inside storage device 206 and flow restrictor 210 trap and prevent the liquid from being pulled to ejector head 220, and more specifically, into ejector 224. Instead, air present in the system is drawn by the vacuum created by ejector 224, continuing to pull liquid from collection device 204.

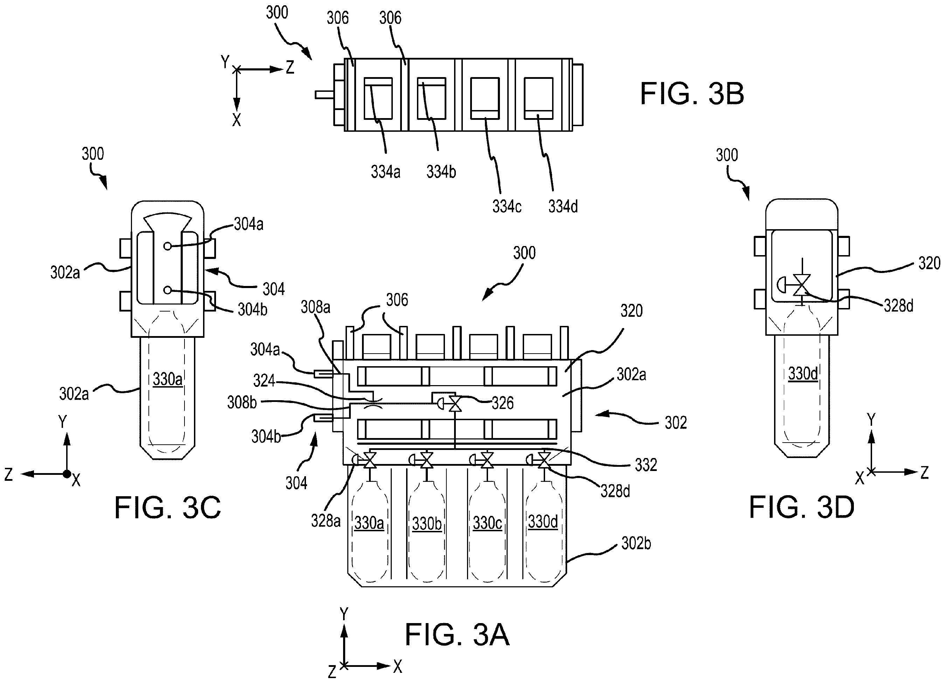

Referring now to FIGS. 3A-3D, a control unit 300 is illustrated, in accordance with various embodiments. Control unit 300 may be an example of control device 208 described above with respect to FIG. 2. Control unit 300 includes similar components to those describe above with respect to control device 208 in FIG. 2, including an ejector head 320, a pressurized gas supply 322, a control inlet port 308a, a control outlet port 308b, an ejector 324, a pressure regulator 326, a first valve 328a, a second valve 328b, a third valve 328c, a first CO₂ cartridge 330a, a second CO₂ cartridge 330b, and a third CO₂ cartridge 330c. These components are described above with respect to FIG. 2, the description of which may not be repeated below. Control unit 300 further includes a housing 302, a fast connector 304, a fourth valve 328d, a fourth CO₂ cartridge 330c, a first toggle 334a, a second toggle 334b, a third toggle 334b, a fourth toggle 334d.

Housing 302 includes an upper portion 302a and a lower portion 302b that may be detachable from upper portion 302a. Upper portion 302a houses ejector head 320 including control inlet port 308a, control outlet port 308b, ejector 324, and pressure regulator 326. Lower portion 302b encloses valves 328a-328d and CO₂ cartridges 330a-330d. In various embodiments, lower portion 302b may be coupled to upper portion 302a using snaps, buttons, friction retainers, or press fitting, among others. In various embodiments, lower portion 302b may be coupled to upper portion 302a by sliding a feature of lower portion 302b into a feature of upper portion 302a, such as rails for example. A high pressure line 332 connects valves 328a-328d to ejector head 320, and more specifically, to pressure regulator 326. Toggles 334a-334d are coupled to upper portion 302a and are configured to engage valves 328a-328d individually. In various embodiments, housing 302 may further include finger guards 306 disposed between and separating each of the toggles 334a-334d.

Fast connector 304 includes a first port 304a and a second port 304b. Fast connector 304 is removable from housing 302 and is configured to quickly attach to and detach from housing 302 at attachment point 336. In various embodiments, fast connector 304 may include grooves that interface with housing 302 to guide fast connector 304 secure fast connector 304 into place. When fast connector 304 is attached to housing 302, first port 304a is aligned with control inlet port 308a and second port 304b is aligned with control outlet port 308b. A hose, tube, or other connector may be connected to first port 304a at one end a storage device (e.g., storage device 206) at an opposite end. In various embodiments, a hose, tube, or other connector, may be connected to second port 304b to control how air is vented from control unit 300.

Each toggle 334a-334d is configured to engage a corresponding valve 328a-328d. Each toggle 334a-334d is configured to toggle between an engaged position and a disengaged position. Each toggle 334a-334d is initialized in the disengaged position (e.g., first toggle 334a and second toggle 334b). Each toggle 334a-334d may be individually flipped, or switched, to the engaged position (e.g., third toggle 334c and fourth toggle 334d). Toggles 334a-334d are configured to provide tactile and/or audible feedback to the user (e.g., pilot 102), such as a click, allowing the user to know the toggle was engaged without looking. Each toggle 334a-334d has a length L1 that allows the user (e.g., pilot 102) to feel and activate, or switch, toggle 334a-334d without looking. Length L1 may be about 0.25 inches (about 0.635 centimeter) to about 1 inch (about 2.54 centimeters), and more specifically, about .4 inches (about 1.02 centimeters) to about .6 inches (about 1.52 centimeters). Flipping toggle 334a-334d from the disengaged position to the engage position opens the corresponding valve 328a-328d.

Opening each valve 328a-328d punctures the corresponding CO₂ cartridge 330a-330d, releasing the pressurized air and activating control unit 300. In various embodiments, valves 328a-328d may be configured to open and stay opened, allowing all, or substantially all, the gas of each CO₂ cartridge 330a-330d to exit. In various embodiments, valves 328a-328d may be configured to open and close, allowing the user (e.g., pilot 102) to the control the gas used from each CO₂ cartridge 330a-330d, allowing for using one or more CO₂ cartridges 330a-330d more than once. Typically, one CO₂ cartridge 330a-330d is used at a time. However, in various embodiments, more than one valve 328a-328d may be opened at one time to increase the pressure and/or the mass flow rate of the pressurized CO₂.

Lower portion 302b may be removed from upper portion 302a to remove and replace spent CO₂ cartridges 330a-330d. After removing lower portion 302b, each CO₂ cartridge 330a-330d is removed and replaced. Each toggle 334a-334d is moved, or switched, back to the disengaged position. Lower portion 302b may then be reconnected to upper portion 302a. In various embodiments, this may be accomplished while control unit 300 is connected to a storage device (e.g., storage device 206).

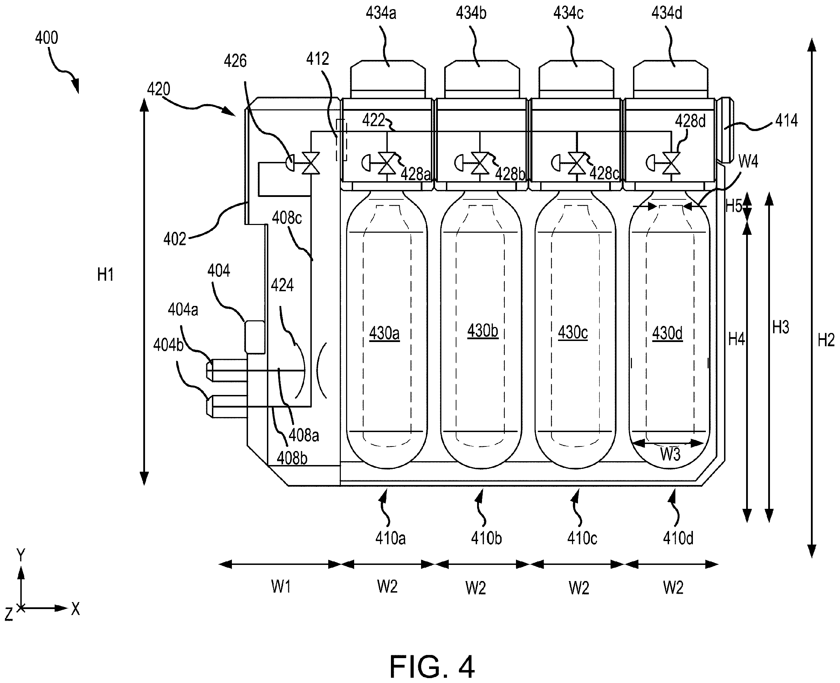

Referring now to FIG. 4, a control unit 400 is illustrated, in accordance with various embodiments. Control unit 400 may be an example of control device 208 described above with respect to FIG. 2. Control unit 400 includes similar components to those describe above with respect to control unit 300 in FIG. 3, including an ejector head 420, a pressurized gas supply 422, a control inlet port 408a, a control outlet port 408b, an ejector 424, a pressure regulator 426, a first valve 428a, a second valve 428b, a third valve 428c, a fourth valve 428d, a first CO₂ cartridge 430a, a second CO₂ cartridge 430b, and a third CO₂ cartridge 430c, a fourth CO₂ cartridge 430d, a first toggle 434a, a second toggle 434b, a third toggle 434c, a fourth toggle 434d, and a fast connector 404. These components are described above with respect to FIG. 3, the description of which may not be repeated below. Control unit 400 further includes an ejector head housing 402, a first cartridge housing 410a, a second cartridge housing 410b, a third cartridge housing 410c, and a fourth cartridge housing 410d.

Ejector head housing 402 encloses control inlet port 408a, control outlet port 408b, a control high pressure port 408c, ejector 424, and pressure regulator 426. Fast connector 404 is removably connected to ejector head housing 402 over control inlet port 408a and control inlet port 408b. Fast connector 404 has a first port 404a and a second port 404b. When fast connector 404 is connected to ejector head housing 402, first port 404a is in communication with control inlet port 408a and second port 404b is in communication with control inlet port 408b.

Ejector head housing 402 has a height H1 (e.g., in the y-direction) and a width W1 (e.g., in the x-direction). Height H1 is about 3.5 inches (about 8.89 centimeters) to about 6 inches (about 15.2 centimeters), and more specifically, about 4 inches (about 10.2 centimeters) to about 5 inches (about 12.7 centimeters). Width W1 is about 1 inch (about 2.54 centimeters) to about 2.5 inches (about 6.35 centimeters), and more specifically, about 1.25 inches (about 3.17 centimeters) to about 2 inches (about 5.08 centimeters).

Each cartridge housing 410a-410d encloses a corresponding valve 428a-428d and a corresponding CO₂ cartridge 430a-430d. Each cartridge housing 410a-410d further includes a corresponding toggle 434a-434d configured to engage the corresponding valve 428a-428d. Cartridge housing 410a-410d has a height H2 (e.g., in the y-direction) and a width W2 (e.g., in the x-direction). Height H2 is about 4 inches (about 10.2 centimeters) to about 7 inches (about 17.8 centimeters), and more specifically, about 4.5 inches (about 11.4 centimeters) to about 6 inches (about 15.2 centimeters). Width W2 is about 1 inch (about 2.54 centimeters) to about 2 inches (about 5.08 centimeters), and more specifically, about 1.25 inches (about 3.17 centimeters) to about 1.75 inches (about 4.44 centimeters).

CO₂ cartridge 430a-430d is sized to fit within cartridge housing 410a-410d having a total height H3, a body height H4, a neck height H5, a body width W3, and a neck width W4. Height H3 is about 3.75 inches (about 9.53 centimeters) to about 5.25 inches (about 13.3 centimeters) and more specifically, about 3.5 inches (about 8.89 centimeters). Body height H4 is about 3.5 inches (about 8.89 centimeters) to about 4.5 inches (about 11.4 centimeters) and more specifically, about 3 inches (about 7.62 centimeters). Neck height H5 is about 0.25 inches (about 0.635 centimeter) to about 0.75 inches (about 1.9 centimeters), and more specifically, about 0.5 inches (about 1.27 centimeters). Body width W3 is about 0.4 inches (about 1.02 centimeters) to about 1 inch (about 2.54 centimeters), and more specifically, about 0.6 inches (about 1.52 centimeters) to about 0.8 inches (about 2.03 centimeters). Neck width W4 is about 0.25 inches (about 0.635 centimeter) to about .5 inches (about 1.27 centimeters), and more specifically, about 0.375 inches (about 0.952 centimeter).

Cartridge housing 410a-410d further includes a first connector 412 and a second connector 414. First connector 412 of each cartridge housing 410a-410d is configured to engage second connector 414 of ejector head housing 402 and/or cartridge housing 410a-410d. In various embodiments, first connector 412 may be press fit onto second connector 414. In various embodiments, first connector 412 may slide onto second connector 414. In various embodiments, an O-ring may be used to provide an air tight seal between first connector 412 and second connector 414. Connections between first connector 412 and second connector 414 forms a high pressure line 432. In various embodiments, high pressure line 432 may be a conduit, a tube, a passageway, or an opening, among others.

This configuration allows control unit 400 to be modular. That is, ejector head housing 402 may be connected to one or more cartridge housings 410a-410d. This allows the user (e.g., pilot 102) to attach as many cartridge housings 410a-410d as will be used during a specific mission. The user may choose different numbers of cartridge housings 410a-410d for size, weight, and/or number of uses anticipated for a mission. Additionally, this allows for reloading control unit 400 by swapping cartridge housings 410a-410d.

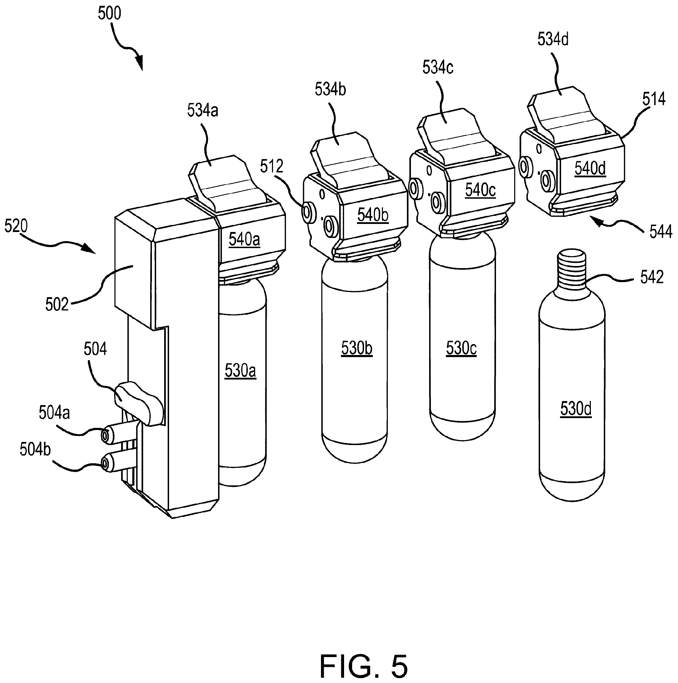

Referring now to FIG. 5, a modular control unit 500 is illustrated in accordance with various embodiments. Modular control unit 500 includes similar components to control unit 400 described above in FIG. 4 including an ejector head housing 502, a fast connector 504, a first port 504a, a second port 504b, a first connector 512, a second connector 514, an ejector head 520, a first pressurized gas source 530a, a second pressurized gas source 530b, and a third pressurized gas source 530c, a fourth pressurized gas source 530d, a first toggle 534a, a second toggle 534b, a third toggle 534c, and a fourth toggle 534d, among others which may not be illustrated, descriptions of which may not be repeated below. Modular control unit 500 further includes a first control head 540a, a second control head 540b, a third control head 540c, and a fourth control head 540d. First control head 540a includes a first valve (e.g., first valve 428a), second control head 540b includes a second valve (e.g., second valve 428b), third control head 540c includes a third valve (e.g., third valve 428c), and fourth control head 540d includes a fourth valve (e.g., fourth valve 428d).

As previously discussed, pressurized gas sources 530a-530d may be any pressurized gas source, including pressurized air cartridge, CO₂ cartridge, and/or nitrogen cartridge, among others. Pressurized gas sources 530a-530d each include a seal to contain the gas therein and prevent from leaking out. The gas contained in each pressurized gas source 530a-530d may be expelled from the respective pressurized gas source 530a-530d in response to the seal being broken, or opened. For simplicity, pressurized gas sources 530a-530d will be referred to as CO₂ cartridges 530a-530d below. Each CO₂ cartridge 530a-530d may include a threaded neck, such as threaded neck 542 illustrated on fourth CO₂ cartridge 530d. Each control head 540a-540d may include a complimentary pressurized gas source input 544 (e.g., a threaded opening) that is configured to receive threaded neck 542 of CO₂ cartridge 530d. In various embodiments, CO₂ cartridges 530a-530d may be configured to be press fit into control heads 540a-540d. That is, CO₂ cartridge 530d may include an indentation, and in various embodiments, an O-ring, instead of threading around the neck (e.g., threaded neck 542) and pressurized gas source input 544 of fourth control head 540d may be configured to receive and seat fourth CO₂ cartridge 530d accordingly.

Each CO₂ cartridge 530a-530d, after being coupled to a respective control head 540a-540d, is in fluid communication with a valve located within each control head 540a-540d. Each valve is in fluid communication with an output connector (e.g., first connector 512) to allow the pressurized gas to flow from CO₂ cartridge (e.g., CO₂ cartridge 530b) to ejector head 520 through the output connector.

Each control head 540a-540d further includes an input connector (e.g., second connector 514) that is configured to receive the output connector of an adjacent control head 540a-540d and pass the pressurized gas from the adjacent control head to ejector head 520. In various embodiments, the flow pressurized gas from the input connector to the output connector bypasses the valve within control head 540a-540d.

Each toggle 534a-534d is coupled to a respective control head 540a-540d. As previously discussed, each toggle (e.g., toggle 534d) is rotational coupled to a respective control head (e.g., fourth control head 540d). Toggle 534d may rotate from a closed, or off, position to an open, or on, position and in so doing puncture the seal of CO₂ cartridge 530d. In various embodiments, the seal may be punctured, or opened, in response to being inserted into fourth control head 540d. The gas is then released from CO₂ cartridge 530d and into fourth control head 540d where the valve controls and directs the pressurized CO₂ toward ejector head 520.

Each output connector (e.g., first connector 512) is configured to mate with, or be coupled to, a respective input connector (e.g., second connector 514) on an adjacent control head. In various embodiments, the input connector and output connector may be press fit together to form a seal and secure a first control head (e.g., first control head 540a) to a second control head (e.g., second control head 540b). In various embodiments, the connection between control heads (e.g., first control head 540a and second control 540b) may be secured using a threaded connection such as, for example, a bolt and nut configuration or a bolt and insert configuration, among others. In various embodiments, there may be a snap coupled around the input connector and output connector that allows the first control head (e.g., first control head 540a) to snap into the second control head (e.g., second control head 540b). In various embodiments, one or more O-rings may be utilized to improve the seal between the adjoined control heads (e.g., first control head 540a and second control head 540b). Additionally, or in the alternative, the O-rings may, in various embodiments, act as a shock absorber or cushion to provide a better connection between the adjoined control heads (e.g., first control head 540a and second control head 540b).

In various embodiments, the adjacent control heads (e.g., first control head 540a and second control head 540b) may be slidably coupled together. In various embodiments, each control head (e.g., first control head 540a and second control head 540b) may include a tongue and groove configuration that is configured to slide into place and secure adjoining control heads (e.g., first control head 540a and second control head 540b) to each other. In various embodiments, each control head may include a set of rails that are configured to interlock and secure the adjoining control heads (e.g., first control head 540a and second control head 540b) to each other. In various embodiments, a latch may be included to prevent the control heads (e.g., first control head 540a and second control head 540b) from separating after being joined.

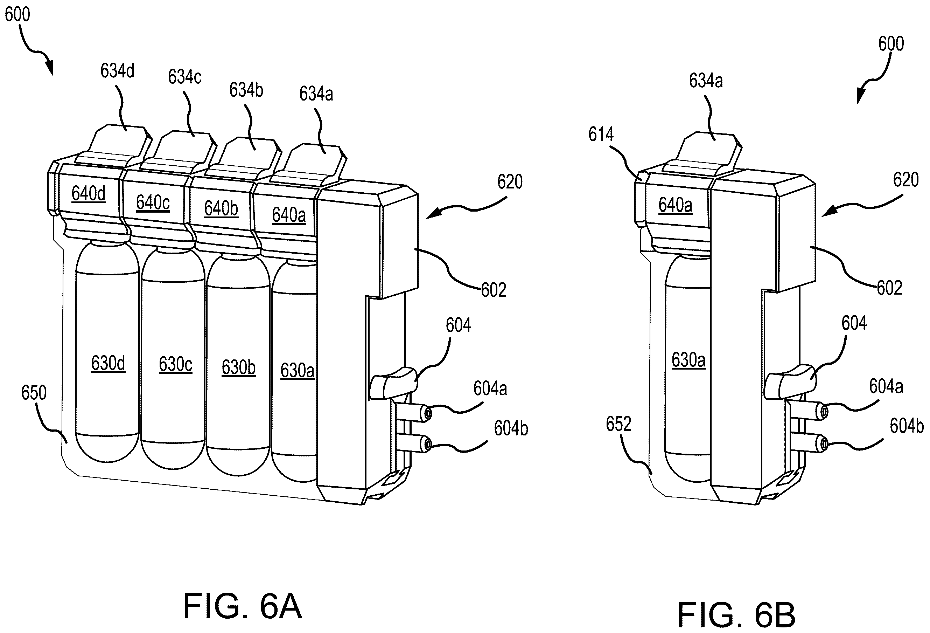

Referring now to FIGS. 6A and 6B, a modular control unit 600 including a housing is illustrated, in accordance with various embodiments. Modular control unit 600 includes similar components to modular control unit 500 described above in FIG. 5 including an ejector head housing 602, a fast connector 604, a first port 604a, a second port 604b, a second connector 614, an ejector head 620, a first pressurized gas source 630a, a second pressurized gas source 630b, and a third pressurized gas source 630c, a fourth pressurized gas source 630d, a first toggle 634a, a second toggle 634b, a third toggle 634c, and a fourth toggle 634d, a first control head 640a, a second control head 640b, a third control head 640c, and a fourth control head 640d, among others which may not be illustrated, descriptions of which may not be repeated below. Modular control unit 600 further includes a housing to secure and support control heads 640a-640d and pressurized gas sources 630a-630d. FIG. 6A illustrates modular control unit 600 including a housing 650 for four pressurized gas sources 630a-630d. FIG. 6B illustrates modular control unit 600 including a modular housing 652 for a single pressurized gas source 630a.

Housing 650 includes a first end 650a and a second end 650b and encloses and secures pressurized gas sources 630a-630d to ejector head 620. In various embodiments, housing 650 may be a single, monolithic component configured to house one or more pressurized gas sources 630a-630d. In various embodiments, first end 650a of housing 650 is configured to engage ejector head 620, and in various embodiments, secured to ejector head 620. In various embodiment, second end 650b is configured to engage second connector 614, and in various embodiments, be secured to second connector 614. is configured to couple to ejector head 620 at a first end and to second connector 614 at a second end.

In various embodiments, modular housing 652 may be configured to house a single pressurized gas source (e.g., first pressurized gas source 630a). Modular housing 652 may be configured to engage and/or interlock with an adjacent modular housing 652. Modular housing has a first end 652a and a second end 652b. First end 652a of a first modular housing 652 is configured to engage ejector head 620 and/or second end 652b of a second modular housing 652. That is, each modular housing 652 is configured to house a single pressurized gas source (e.g., first pressurized gas source 630a) and is further configured to be secured to an adjacent modular housing 652.

As disclosed herein, housing 650, or modular housing 652, provide additional support and security for pressurized gas sources 630a-630d. The additional support prevents one or more pressurized gas sources 630a-630d from separating from a respective control head 640a-640d and breaking open the one or more pressurized gas sources 630a-630d. Housing 650, and modular housing 652, provide added comfort of use and ease of transport by enclosing the individual components of modular control unit 600. Furthermore, housing 650 and modular housing 652 may, in various embodiments, provide a thermal barrier between the pressurized gas source and the user of modular control unit 600. Accordingly, housing 650 and/or modular housing 652 may, in various embodiments, further include an insulator such as rubber, silicone, cork, wood, plastic, or a cloth material, among others.

Benefits, other advantages, and solutions to problems have been described herein with regard to specific embodiments. Furthermore, the connecting lines shown in the various figures contained herein are intended to represent exemplary functional relationships and/or physical couplings between the various elements. It should be noted that many alternative or additional functional relationships or physical connections may be present in a practical system. However, the benefits, advantages, solutions to problems, and any elements that may cause any benefit, advantage, or solution to occur or become more pronounced are not to be construed as critical, required, or essential features or elements of the disclosure. The scope of the disclosure is accordingly to be limited by nothing other than the appended claims, in which reference to an element in the singular is not intended to mean "one and only one" unless explicitly so stated, but rather "one or more." Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C. Different cross-hatching is used throughout the figures to denote different parts but not necessarily to denote the same or different materials.

Systems, methods, and apparatus are provided herein. In the detailed description herein, references to "one embodiment," "an embodiment," "various embodiments," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

Numbers, percentages, or other values stated herein are intended to include that value, and also other values that are about or approximately equal to the stated value, as would be appreciated by one of ordinary skill in the art encompassed by various embodiments of the present disclosure. A stated value should therefore be interpreted broadly enough to encompass values that are at least close enough to the stated value to perform a desired function or achieve a desired result. The stated values include at least the variation to be expected in a suitable industrial process, and may include values that are within 5% of a stated value. Additionally, the terms "substantially," "about" or "approximately" as used herein represent an amount close to the stated amount that still performs a desired function or achieves a desired result. For example, the term "substantially," "about" or "approximately" may refer to an amount that is within 5% of a stated amount or value.

Furthermore, no element, component, or method step in the present disclosure is intended to be dedicated to the public regardless of whether the element, component, or method step is explicitly recited in the claims. As used herein, the terms "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus.

Finally, it should be understood that any of the above-described concepts can be used alone or in combination with any or all of the other above-described concepts. Although various embodiments have been disclosed and described, one of ordinary skill in this art would recognize that certain modifications would come within the scope of this disclosure. Accordingly, the description is not intended to be exhaustive or to limit the principles described or illustrated herein to any precise form. Many modifications and variations are possible in light of the above teaching.

## Claims

1. A control head (540a-540d) for a modular control unit of a urinary relief system, comprising:
a pressurized gas source input;
a toggle (534a-534d) configured to activate a pressurized gas source (530a-530d) coupled to the pressurized gas source input;
a first connector (512) fluidly coupled to the pressurized gas source input and configured to allow a first gas from the pressurized gas source input to flow through the control head;
**characterized by**
a second connector (514) fluidly coupled to the first connector and configured to allow a second gas to pass through the control head;
wherein the pressurized gas source input is configured to receive a pressurized air cartridge via at least one of a threaded opening or a press fit connection.

2. The control head of the modular control unit of the urinary relief system of claim 1, further comprising:
a valve coupled to the pressurized gas source input, wherein the first connector (512) is fluidly coupled to the pressurized gas source input through the valve; and optionally wherein the toggle (534a-534d) is further configured to open the valve.

3. The control head of the modular control unit of the urinary relief system of claim 1 or 2, further comprising:
a first connector on the first connector (512); and
a second connector on the second connector (514), the second connector configured to engage the first connector.

4. The control head of the modular control unit of the urinary relief system of claim 3,
wherein the first connector (512) includes a first rail and the second connector (514) includes a second rail that is configured to engage the first rail; or
wherein the first connector includes a first threading and the second connector includes a second threading that is configured to engage the first threading; or
wherein the first connector includes a first snap connector and the second connector includes a second snap connector that is configured to engage the first snap connector.

5. A modular control unit for a urinary relief system, comprising:
a first pressurized gas source (530a);
a pressure regulator (226) configured to regulate a pressure of the first pressurized gas source;
an ejector (224) coupled to the pressure regulator and an output;
an ejector head housing the pressure regulator and the ejector;
a first control head (540a) coupled to the ejector head housing, the first control head including:
a first pressurized gas source input configured to receive the first pressurized gas source;
a first toggle (534a) configured to activate the first pressurized gas source;
a first connector (512) fluidly coupled to the first pressurized gas source input and fluidly coupled to the ejector head housing, the first connector configured to allow a first gas from the first pressurized gas source input to flow through the first control head and into the ejector head housing; and
a second connector (514) fluidly coupled to the first connector and configured to allow a second gas to pass through the first control head;
wherein the pressurized gas source input is configured to receive the first pressurized gas source via at least one of a threaded opening or a press fit connection.

6. The modular control unit for the urinary relief system of claim 5, further comprising:
a second pressurized gas source (530b); and
a second control head (540b) coupled to the first control head (540a), the second control head including:
a second pressurized gas source input configured to receive the second pressurized gas source;
a second toggle (534b) configured to activate the second pressurized gas source; and
a first connector fluidly coupled to the second pressurized gas source input and fluidly coupled to the first control head, the first connector configured to allow the second gas from the second pressurized gas source input to flow through the second control head and into the first control head through the second connector of the first control head.

7. The modular control unit for the urinary relief system of claim 6, wherein the second connector (514) of the first control head is configured to receive the first connector of the second control head.

8. The modular control unit for the urinary relief system of claim 7, wherein the second connector (514) of the first control head includes a first rail and the first connector of the second control head includes a second rail that is configured to engage the first rail.

9. The modular control unit for the urinary relief system of claim 6, 7 or 8,
further comprising:
a housing coupled to the ejector head housing and the second control head, the housing being configured to house the first pressurized gas source (530a) and the second pressurized gas source (530b);
or
further comprising:
a first housing coupled to the ejector head housing and the first control head (540a), the first housing configured to house the first pressurized gas source (530a); and
a second housing coupled to the first housing and the second control head (540b), the second housing configured to house the second pressurized gas source (530b).

10. The modular control unit for the urinary relief system of any of claims 5 to 9, wherein the first control head (540a) further comprises:
a first valve coupled to the first pressurized gas source input, wherein the first connector (512) is fluidly coupled to the first pressurized gas source input through the first valve, wherein the first toggle (534a) is further configured to open the first valve.

11. A urinary relief system, comprising:
a human interface device (104);
a storage device (106) coupled to the human interface device; and
the modular control unit (108) of any of claims 5-10 coupled to the storage device.

12. The urinary relief system of claim 11, when dependent on claim 6, wherein the first control head (540a) is slidably coupled to the second control head (540b).

## Patentansprüche

1. Steuerkopf (540a-540d) für eine modulare Steuereinheit eines Harnentlastungssystems, umfassend:
einen Druckgasquelleneingang;
einen Kippschalter (534a-534d), der konfiguriert ist, um eine Druckgasquelle (530a-530d) zu aktivieren, die mit dem Druckgasquelleneingang gekoppelt ist;
einen ersten Verbinder (512), der fluidisch mit dem Druckgasquelleneingang gekoppelt und konfiguriert ist, um zu ermöglichen, dass ein erstes Gas von dem Druckgasquelleneingang durch den Steuerkopf strömt;
**gekennzeichnet durch**
einen zweiten Verbinder (514), der fluidisch mit dem ersten Verbinder gekoppelt und konfiguriert ist, um zu ermöglichen, dass ein zweites Gas durch den Steuerkopf passiert;
wobei der Druckgasquelleneingang konfiguriert ist, um eine Druckluftpatrone über mindestens eine von einer Gewindeöffnung oder einer Presspassungsverbindung aufzunehmen.

2. Steuerkopf der modularen Steuereinheit des Harnentlastungssystems nach Anspruch 1,
ferner umfassend:
ein Ventil, das mit dem Druckgasquelleneingang gekoppelt ist, wobei der erste Verbinder (512) durch das Ventil fluidisch mit dem Druckgasquelleneingang gekoppelt ist; und wobei der Kippschalter (534a-534d) optional ferner konfiguriert ist, um das Ventil zu öffnen.

3. Steuerkopf der modularen Steuereinheit des Harnentlastungssystems nach Anspruch 1 oder 2,
ferner umfassend:
einen ersten Verbinder auf dem ersten Verbinder (512); und
einen zweiten Verbinder auf dem zweiten Verbinder (514), wobei der zweite Verbinder konfiguriert ist, um mit dem ersten Verbinder in Eingriff zu kommen.

4. Steuerkopf der modularen Steuereinheit des Harnentlastungssystems nach Anspruch 3, wobei der erste Verbinder (512) eine erste Schiene beinhaltet und der zweite Verbinder (514) eine zweite Schiene beinhaltet, die konfiguriert ist, um mit der ersten Schiene in Eingriff zu kommen; oder
wobei der erste Verbinder ein erstes Gewinde beinhaltet und der zweite Verbinder ein zweites Gewinde beinhaltet, das konfiguriert ist, um mit dem ersten Gewinde in Eingriff zu kommen; oder
wobei der erste Verbinder einen ersten Schnappverbinder beinhaltet und der zweite Verbinder einen zweiten Schnappverbinder beinhaltet, der konfiguriert ist, um mit dem ersten Schnappverbinder in Eingriff zu kommen.

5. Modulare Steuereinheit für ein Harnentlastungssystem, umfassend:
eine erste Druckgasquelle (530a);
einen Druckregler (226), der konfiguriert ist, um einen Druck der ersten Druckgasquelle zu regeln;
einen Ejektor (224), der mit dem Druckregler und einem Ausgang gekoppelt ist;
einen Ejektorkopf, der den Druckregler und den Ejektor beherbergt;
einen ersten Steuerkopf (540a), der mit dem Ejektorkopfgehäuse gekoppelt ist, wobei der erste Steuerkopf Folgendes beinhaltet:
einen ersten Druckgasquelleneingang, der konfiguriert ist, um die erste Druckgasquelle aufzunehmen;
einen ersten Kippschalter (534a), der konfiguriert ist, um die erste Druckgasquelle zu aktivieren;
einen ersten Verbinder (512), der fluidisch mit dem ersten Druckgasquelleneingang gekoppelt und fluidisch mit dem Ejektorkopfgehäuse gekoppelt ist, wobei der erste Verbinder konfiguriert ist, um zu ermöglichen, dass ein erstes Gas von dem ersten Druckgasquelleneingang durch den ersten Steuerkopf und in das Ejektorkopfgehäuse strömt; und
einen zweiten Verbinder (514), der fluidisch mit dem ersten Verbinder gekoppelt und konfiguriert ist, um zu ermöglichen, dass ein zweites Gas durch den ersten Steuerkopf passiert;
wobei der Druckgasquelleneingang konfiguriert ist, um die erste Druckgasquelle über mindestens eine von einer Gewindeöffnung oder einer Presspassungsverbindung aufzunehmen.

6. Modulare Steuereinheit für das Harnentlastungssystem nach Anspruch 5, ferner umfassend:
eine zweite Druckgasquelle (530b); und
einen zweiten Steuerkopf (540b), der mit dem ersten Steuerkopf (540a) gekoppelt ist, wobei der zweite Steuerkopf Folgendes beinhaltet:
einen zweiten Druckgasquelleneingang, der konfiguriert ist, um die zweite Druckgasquelle aufzunehmen;
einen zweiten Kippschalter (534b), der konfiguriert ist, um die zweite Druckgasquelle zu aktivieren; und
einen ersten Verbinder, der fluidisch mit dem zweiten Druckgasquelleneingang gekoppelt und fluidisch mit dem ersten Steuerkopf gekoppelt ist, wobei der erste Verbinder konfiguriert ist, um zu ermöglichen, dass das zweite Gas von dem zweiten Druckgasquelleneingang durch den zweiten Steuerkopf und in den ersten Steuerkopf durch den zweiten Verbinder des ersten Steuerkopfes strömt.

7. Modulare Steuereinheit für das Harnentlastungssystem nach Anspruch 6, wobei der zweite Verbinder (514) des ersten Steuerkopfes konfiguriert ist, um den ersten Verbinder des zweiten Steuerkopfes aufzunehmen.

8. Modulare Steuereinheit für das Harnentlastungssystem nach Anspruch 7, wobei der zweite Verbinder (514) des ersten Steuerkopfes eine erste Schiene beinhaltet und der erste Verbinder des zweiten Steuerkopfes eine zweite Schiene beinhaltet, die konfiguriert ist, um mit der ersten Schiene in Eingriff zu kommen.

9. Modulare Steuereinheit für das Harnentlastungssystem nach Anspruch 6, 7 oder 8,
ferner umfassend:
ein Gehäuse, das mit dem Ejektorkopfgehäuse und dem zweiten Steuerkopf gekoppelt ist, wobei das Gehäuse konfiguriert ist, um die erste Druckgasquelle (530a) und die zweite Druckgasquelle (530b) aufzunehmen;
oder
ferner umfassend:
ein erstes Gehäuse, das mit dem Ejektorkopfgehäuse und dem ersten Steuerkopf (540a) gekoppelt ist, wobei das erste Gehäuse konfiguriert ist, um die erste Druckgasquelle (530a) aufzunehmen; und
ein zweites Gehäuse, das mit dem ersten Gehäuse und dem zweiten Steuerkopf (540b) gekoppelt ist, wobei das zweite Gehäuse konfiguriert ist, um die zweite Druckgasquelle (530b) aufzunehmen.

10. Modulare Steuereinheit für das Harnentlastungssystem nach einem der Ansprüche 5 bis 9, wobei der erste Steuerkopf (540a) ferner Folgendes umfasst:
ein erstes Ventil, das mit dem ersten Druckgasquelleneingang gekoppelt ist, wobei der erste Verbinder (512) durch das erste Ventil fluidisch mit dem ersten Druckgasquelleneingang gekoppelt ist, wobei der erste Kippschalter (534a) ferner konfiguriert ist, um das erste Ventil zu öffnen.

11. Harnentlastungssystem, umfassend:
ein Mensch-Schnittstellenvorrichtung (104);
eine Speichervorrichtung (106), die mit der Mensch-Schnittstellenvorrichtung gekoppelt ist; und
die modulare Steuereinheit (108) nach einem der Ansprüche 5-10, die mit der Speichervorrichtung gekoppelt ist.

12. Harnentlastungssystem nach Anspruch 11, wenn abhängig von Anspruch 6, wobei der erste Steuerkopf (540a) verschiebbar mit dem zweiten Steuerkopf (540b) gekoppelt ist.

## Revendications

1. Tête de commande (540a-540d) pour une unité de commande modulaire d'un système de soulagement urinaire, comprenant :
une entrée de source de gaz sous pression ;
une bascule (534a-534d) configurée pour activer une source de gaz sous pression (530a-530d) accouplée à l'entrée de source de gaz sous pression ;
un premier connecteur (512) accouplé fluidiquement à l'entrée de source de gaz sous pression et configuré pour permettre à un premier gaz à partir de l'entrée de source de gaz sous pression de s'écouler à travers la tête de commande ;
**caractérisée par**
un second connecteur (514) accouplé fluidiquement au premier connecteur et configuré pour permettre à un second gaz de passer à travers la tête de commande ;
dans laquelle l'entrée de source de gaz sous pression est configurée pour recevoir une cartouche d'air sous pression par l'intermédiaire d'au moins l'un d'une ouverture filetée ou d'un raccord à ajustement serré.

2. Tête de commande de l'unité de commande modulaire du système de soulagement urinaire selon la revendication 1,
comprenant en outre :
une vanne accouplée à l'entrée de source de gaz sous pression, dans laquelle le premier connecteur (512) est accouplé fluidiquement à l'entrée de source de gaz sous pression à travers la vanne ; et optionnellement, dans laquelle la bascule (534a-534d) est en outre configurée pour ouvrir la vanne.

3. Tête de commande de l'unité de commande modulaire du système de soulagement urinaire selon la revendication 1 ou 2, comprenant en outre :
un premier connecteur sur le premier connecteur (512) ; et
un second connecteur sur le second connecteur (514), le second connecteur étant configuré pour venir en prise avec le premier connecteur.

4. Tête de commande de l'unité de commande modulaire du système de soulagement urinaire selon la revendication 3, dans laquelle le premier connecteur (512) comporte un premier rail et le second connecteur (514) comporte un second rail qui est configuré pour venir en prise avec le premier rail ; ou
dans laquelle le premier connecteur comporte un premier filetage et le second connecteur comporte un second filetage qui est configuré pour venir en prise avec le premier filetage ; ou
dans laquelle le premier connecteur comporte un premier connecteur à encliquetage et le second connecteur comporte un second connecteur à encliquetage qui est configuré pour venir en prise avec le premier connecteur à encliquetage.

5. Unité de commande modulaire pour un système de soulagement urinaire, comprenant :
une première source de gaz sous pression (530a) ;
un régulateur de pression (226) configuré pour réguler une pression de la première source de gaz sous pression ;
un éjecteur (224) accouplé au régulateur de pression et à une sortie ;
une tête d'éjecteur logeant le régulateur de pression et l'éjecteur ;
une première tête de commande (540a) accouplée au boîtier de tête d'éjecteur, la première tête de commande comportant :
une première entrée de source de gaz sous pression configurée pour recevoir la première source de gaz sous pression ;
une première bascule (534a) configurée pour activer la première source de gaz sous pression ;
un premier connecteur (512) accouplé fluidiquement à la première entrée de source de gaz sous pression et accouplé fluidiquement au boîtier de tête d'éjecteur, le premier connecteur étant configuré pour permettre à un premier gaz à partir de la première entrée de source de gaz sous pression de s'écouler à travers la première tête de commande et dans le boîtier de tête d'éjecteur ; et
un second connecteur (514) accouplé fluidiquement au premier connecteur et configuré pour permettre à un second gaz de passer à travers la première tête de commande ;
dans laquelle l'entrée de source de gaz sous pression est configurée pour recevoir la première source de gaz sous pression par l'intermédiaire d'au moins l'un d'une ouverture filetée ou d'un raccord à ajustement serré.

6. Unité de commande modulaire pour le système de soulagement urinaire selon la revendication 5, comprenant en outre :
une seconde source de gaz sous pression (530b) ; et
une seconde tête de commande (540b) accouplée à la première tête de commande (540a), la seconde tête de commande comportant :
une seconde entrée de source de gaz sous pression configurée pour recevoir la seconde source de gaz sous pression ;
une seconde bascule (534b) configurée pour activer la seconde source de gaz sous pression ; et
un premier connecteur accouplé fluidiquement à la seconde entrée de source de gaz sous pression et accouplé fluidiquement à la première tête de commande, le premier connecteur étant configuré pour permettre au second gaz à partir de la seconde entrée de source de gaz sous pression de s'écouler à travers la seconde tête de commande et dans la première tête de commande à travers le second connecteur de la première tête de commande.

7. Unité de commande modulaire pour le système de soulagement urinaire selon la revendication 6, dans laquelle le second connecteur (514) de la première tête de commande est configuré pour recevoir le premier connecteur de la seconde tête de commande.

8. Unité de commande modulaire pour le système de soulagement urinaire selon la revendication 7, dans laquelle le second connecteur (514) de la première tête de commande comporte un premier rail et le premier connecteur de la seconde tête de commande comporte un second rail qui est configuré pour venir en prise avec le premier rail.

9. Unité de commande modulaire pour le système de soulagement urinaire selon la revendication 6, 7 ou 8,
comprenant en outre :
un boîtier accouplé au boîtier de tête d'éjecteur et à la seconde tête de commande, le boîtier étant configuré pour loger la première source de gaz sous pression (530a) et la seconde source de gaz sous pression (530b) ;
ou
comprenant en outre :
un premier boîtier accouplé au boîtier de tête d'éjecteur et à la première tête de commande (540a), le premier boîtier configuré pour loger la première source de gaz sous pression (530a) ; et
un second boîtier accouplé au premier boîtier et à la seconde tête de commande (540b), le second boîtier étant configuré pour loger la seconde source de gaz sous pression (530b).

10. Unité de commande modulaire pour le système de soulagement urinaire selon l'une quelconque des revendications 5 à 9, dans laquelle la première tête de commande (540a) comprend en outre :
une première vanne accouplée à la première entrée de source de gaz sous pression, dans laquelle le premier connecteur (512) est accouplé fluidiquement à la première entrée de source de gaz sous pression à travers la première vanne, dans laquelle la première bascule (534a) est en outre configurée pour ouvrir la première vanne.

11. Système de soulagement urinaire, comprenant :
un dispositif d'interface humaine (104) ;
un dispositif de stockage (106) accouplé au dispositif d'interface humaine ; et
l'unité de commande modulaire (108) selon l'une quelconque des revendications 5 à 10 accouplée au dispositif de stockage.

12. Système de soulagement urinaire selon la revendication 11, lorsqu'elle dépend selon la revendication 6, dans lequel la première tête de commande (540a) est accouplée de manière coulissante à la seconde tête de commande (540b).
